**Europäisches Patentamt**

**European Patent Office**

**Office européen des brevets**

(19)

(11) Veröffentlichungsnummer : **0 480 238 B1**

(12)

# EUROPÄISCHE PATENTSCHRIFT

(45) Veröffentlichungstag der Patentschrift :
**20.07.94 Patentblatt 94/29**

(51) Int. Cl.⁵ : **A61K 6/10, C08L 83/07**

(21) Anmeldenummer : **91116251.9**

(22) Anmeldetag : **24.09.91**

(54) **Hydrophilierte Abformmassen.**

(30) Priorität : **06.10.90 DE 4031759**

(43) Veröffentlichungstag der Anmeldung :
**15.04.92 Patentblatt 92/16**

(45) Bekanntmachung des Hinweises auf die
Patenterteilung :
**20.07.94 Patentblatt 94/29**

(84) Benannte Vertragsstaaten :
**AT BE CH DE DK ES FR GB GR IT LI LU NL SE**

(56) Entgegenhaltungen :
**EP-A- 0 154 922**
**EP-A- 0 219 660**
**EP-A- 0 268 347**
**DE-A- 3 721 784**
**DE-A- 3 931 416**

(73) Patentinhaber : **BAYER AG**
**D-51368 Leverkusen (DE)**

(72) Erfinder : **Voigt, Reiner, Dr.**
**An der Lichtenburg 4**
**W-5090 Leverkusen (DE)**
Erfinder : **Schwabe, Peter, Dr.**
**Dudweiler Strasse 17**
**W-5090 Leverkusen (DE)**
Erfinder : **Knispel, Gottfried, Dr.**
**Richard-Wagner-Strasse 3**
**W-5090 Leverkusen (DE)**
Erfinder : **Flindt, Roland, Dr.**
**Doerperhofstrasse 31**
**W-4150 Krefeld (DE)**

**Beschreibung**

Die vorliegende Erfindung betrifft hydrophilierte Abform- bzw. Dubliermassen auf Polysiloxanbasis, die insbesondere im Dentalbereich Anwendung finden sowie ein Verfahren zu deren Herstellung.

Die vorliegende Erfindung betrifft insbesondere additionsvernetzende Vinylsilicon-Pasten zum Herstellen genauer Abformungen bezahnter, teilbezahnter und unbezahnter Kiefer sowie von Gipsmodellen. Hierbei handelt es sich um ein kaltvulkanisierendes Zweikomponenten-Siliconkautschuksystem (RTV), bei dem die vernetzerhaltige Basispaste mit einer Katalysatorpaste vermengt wird und bei Raumtemperatur vernetzt.

Derartige Systeme sind an sich bekannt (vgl. z.B. R.G. Graig, Restorative Dental Materials, The C.V. Moosbe-Comp., St. Louis, 1980, S. 195 ff).

Im allgemeinen bestehen diese Massen aus einer Siliconöl, Füllstoff und Vernetzer enthaltenden Basispaste und einer Katalysatorpaste aus Siliconöl, Füllstoff und Katalysator. Für die verschiedenen Abformmethoden werden die Massen in verschiedenen Viskositätsklassen angeboten, so beispielsweise die knetbaren und hochviskosen Massen für die Löffel- und die mittel- und niedrig-viskosen Massen vorzugsweise für die Spritzenapplikation.

Bei Anwendung werden äquivalente Mengen der Basis- und Katalysatorpaste, gewichts- oder volumenmäßig vorzugsweise 1:1, zu einer homogenen Masse gemischt und mittels Spritze und/oder Abformlöffel auf die abzuformende Kieferpartie appliziert. Nach dem Vernetzen durch eine Polyadditionsreaktion wird die gummiartige Abformung aus dem Mund des Patienten entfernt und anschließend mit einer wässrigen Aufschlämmung von Modellgips ausgegossen. Nach dem Erhärten des Gipses erhält man die präzise Wiedergabe der Kiefersituation.

Die additionsvernetzenden Siliconabformmassen haben zwar eine hervorragende Dimensionsstabilität, jedoch sind sie hydrophob. So kann es bei der Applikation der gemischten Abformmasse auf die feuchte Oberfläche von Zähnen und Zahnfleisch zu einem Fließstau oder in den Zahnfleischtaschen durch noch vorhandene Flüssigkeitsreste zu einer ungenügenden Verdrängung dieser kommen, so daß die vernetzte Abformung dann Fehler aufweist. Zum anderen können beim Ausgießen der hydrophoben Siliconabformung mit der wässrigen Gipsaufschlämmung durch die Unverträglichkeit der Oberflächenmedien Luftbläschen eingeschlossen werden; ein fehlerhaftes Gipsmodell ist die Folge.

Durch Hydrophilierung der Siliconabformmassen kann man diese Fehlerquellen weitgehend beseitigen. So werden in US 4.691.039 und US 4.752.633 ethoxylierte Siloxane, in US 4.657.959 Polyethersilicone und fluorhaltige Siliconverbindungen als Zusätze zu den Siliconmassen beschrieben. Laut DE-A-3.721.784 können wasserlösliche und wenig lösliche Proteine in Verbindung mit ethoxylierten Siloxanen, Fettalkoholen, Fettsäuren, Estern und Bienenwachsderivaten sowie fluorhaltige Verbindungen eingesetzt werden. EP-A-0.231.420 beschreibt Zusätze von wasserab-/adsorbierenden Füllstoffen wie $CaSO_4 \cdot 1/2\ H_2O$, $CaCl_2$, $K_2SO_4$, Zeolith und Molekularsieben in Verbindung mit Silikopolyether. US 4.782.101 und EP-A-0.268.347 geben additionsvernetzende Siliconabformmassen an, die als Zusätze Polyol-Fettsäureester und ethoxylierte Ester sowie Platinschwarz enthalten. Letzteres dient als Wasserstoffadsorber, da durch eine Reaktion des SiH-gruppenhaltigen Vernetzers mit den OH-Gruppen des Hydrophilierungsmittels beim Ausgießen der vernetzten Abformung mit der wässrigen Gipsaufschlämmung Wasserstoff entstehen kann und diese Gasbläschen zu einem fehlerhaften Gipsmodell führen.

Die vorliegende Erfindung betrifft hydrophilierte additionsvernetzende Siliconabformmassen mit Zusätzen von ausgesuchten ethoxylierten Fettalkoholen, die auch ohne Zusatz von wasserlöslichen oder wenig löslichen Proteinen oder wasserad-/absorbierenden Füllstoffen einen deutlich gesenkten Wasserbenetzungswinkel aufweisen und ohne Platinschwarzzusatz ein einwandfreies Gipsmodell liefern.

Gegenstand der vorliegenden Erfindung sind somit hydrophilierte, bei Umgebungstemperatur additionsvernetzende Abformmassen auf Polysiloxanbasis enthaltend

(a) Organopolysiloxane mit mindestens zwei Vinylgruppen im Molekül,
(b) gegebenenfalls Organopolysiloxane ohne reaktive Gruppen,
(c) Organohydrogenpolysiloxane mit zwei oder mehr Si-H-Gruppen im Molekül,
(d) Katalysator,
(e) Füllstoffe sowie gegebenenfalls weitere übliche Zusatz-, Hilfs- und Farbstoffe
(f) speziellen alkoxylierten Fettalkohol und/oder einen methylierten oder acylierten alkoxylierten Fettalkohol,

dadurch gekennzeichnet, daß sie frei von Platinschwarz sind.

Sowohl die Basis- als auch die Katalysatorpaste werden durch Vermischung der entsprechenden Komponenten (a) bis (f) hergestellt, wobei die Basispaste alle Bestandteile außer (d) und die Katalysatorpaste alle Bestandteile außer (c) enthalten kann. Die für die gewünschte Anwendung optimale Rezeptur kann durch einfache Vorversuche ermittelt werden.

Die erfindungsgemäß als Hydrophilierungsmittel (f) eingesetzten speziellen alkoxylierten Fettalkohole und acylierte alkoxylierten Fettalkohole sind gerad- und verzweigtkettige Alkohole von $C_{10}$ bis $C_{16}$, vorzugsweise $C_{12}$ bis $C_{14}$, welche mit 2 bis 10 Mol Alkylenoxid, bevorzugt mit 2 bis 10 Mol Ethylen- oder Propylenoxid, besonders bevorzugt 4 bis 7 Mol Ethylenoxid, umgesetzt und gegebenenfalls anschließend mit einer $C_2$- bis $C_4$-Monocarbonsäure, vorzugsweise Essigsäure, verestert oder methyliert wurden.

Als erfindungsgemäße Hydrophilierungsmittel sind beispielsweise genannt:

$C_{12}H_{25}O-(CH_2-CH_2-O)_5CH_3$

$C_{13}H_{27}O-(CH_2-CH_2-O)_6CH_3$

$C_{14}H_{29}O-(CH_2-CH_2-O)_7CH_3$

$C_{12}H_{25}O-(CH_2-CH_2-O)_5H$

$C_{13}H_{27}O-(CH_2-CH_2-O)_6H$

$C_{14}H_{29}O-(CH_2-CH_2-O)_7H$

$C_{12}H_{25}O-(CH_2-CH_2-O)_5-CO-CH_3$

$C_{13}H_{27}O-(CH_2-CH_2-O)_6-CO-CH_3$

$C_{14}H_{29}O-(CH_2-CH_2-O)_5-CO-CH_3$

und Mischungen hieraus

Diese Hydrophilierungsmittel werden in Mengen von 0,3 bis 5,0 Gew.%, vorzugsweise 0,5 bis 3,0 Gew.%, bezogen auf die Gesamtmischung Basis- und Katalysatorpaste, entweder der Basispaste allein oder anteilig der Basis- und Katalysatorpaste zugesetzt.

Als Ausgangsstoffe für die erfindungsgemäßen Massen sind folgende Materialien geeignet:

Bei dem Siliconöl (a) handelt es sich um ein Polydimethylsiloxan mit ungesättigten Kohlenwasserstoff-Gruppen, vorzugsweise Vinylgruppen an mindestens zwei Siliciumatomen, dessen Viskosität im Bereich von 500 bis 200000 mPa·s bei 20°C liegen kann, je nach gewünschter Viskosität der formulierten Pasten.

Die Siliconöle (b) sind trimethylsiloxy-endgestoppte Polydimethylsiloxane mit einer Viskosität von 50 bis 2000 mPa·s bei 20°C.

Der Vernetzer (c) ist ein Polydimethylsiloxan, welches in seinem Molekül Wasserstoffatome an mindestens zwei Siliciumatomen aufweist.

Bei dem Katalysator (d) handelt es sich bevorzugt um einen Platinkomplex, der aus Hexachloroplatin-(IV)-säure hergestellt wurde. Auch diese Verbindungen sind an sich bekannt. Geeignet sind auch andere Platinverbindungen, die die Additionsvernetzungsreaktion beschleunigen. Gut geeignet sind z.B. Platin-Siloxan-Komplexe, wie sie z.B. in US-PS 3 715 334, US-PS 3 775 352 und US-PS 3 814 730 beschrieben sind.

Unter den Füllstoffen (e) versteht man Quarz- und Cristobalitmehle, Calciumsulfat. Calciumcarbonat, Diatomeenerde, gefälltes und pyrogen hergestelltes Siliciumdioxid mit unbeladener und beladener Oberfläche.

Farbstoffe werden zur Unterscheidung der Basis- und Katalysatorpaste und zur Mischkontrolle eingesetzt. Anorganische und organische Farbpigmente werden üblicherweise eingesetzt.

Die erfindungsgemäßen hydrophilierten Abformmassen weisen im angemischten pastösen Zustand eine gute Affinität zu der feuchten Zahnhartsubstanz und dem feuchten Zahnfleisch auf. Zum anderen können die vernetzten Abformungen in der üblichen Zeit von 30 Minuten nach Herausnahme des Abdruckes aus dem Mund des Patienten mit der wässrigen Gipsaufschlämmung ausgegossen werden ohne daß durch Wasserstoffentwicklung das Gipsmodell verfälscht wird.

Die nachfolgenden Beispiele erläutern die Erfindung, wobei die Rezepturen der mittelviskosen additionsvernetzenden Siliconabformmassen stellvertretend auch für niedrig- und hochviskose und knetbare Abformmassen sowie für niedrigviskose Dubliermassen stehen. Die angegebenen Teile sind Gewichtsteile.

Beispiel 1 (Vergleich)

In einem Kneter wird eine Basispaste hergestellt durch Mischen von 290 Teilen vinylendgestopptem Polydimethylsiloxan mit einer Viskosität von 10000 mPa·s bei 23°C, 180 Teilen SiH-gruppenhaltigem Polydimethylsiloxan mit einer Viskosität von 300 mPa·s bei 23°C, 515 Teilen Quarzfeinstmehl, 10 Teilen hydriertes Rizinusöl mit einem Schmelzpunkt von 85°C und 5 Teilen anorganisches Farbpigment. Während des Mischprozeßes wird die Paste auf eine Temperatur von 95°C gebracht und anschließend auf Raumtemperatur abgekühlt.

Die Katalysatorpaste wird in einem Kneter hergestellt durch Vermischen von 463 Teilen vinylendgestopptem Polydimethylsiloxan mit einer Viskosität von 5000 mPa·s bei 23 °C, 525 Teilen Quarzfeinstmehl und 10 Teilen hydriertes Rizinusöl mit einem Schmelzpunkt von 85 °C. Die Paste wird unter Mischen auf eine Temperatur von 95 °C aufgeheizt, auf Raumtemperatur abgekühlt und anschließend mit 2 Teilen eines Komplexes aus Platin und Divinyltetramethyldisiloxan versetzt.

Beispiele 2-7 (erfindungsgemäß)

Zusammensetzung und Herstellung der beiden Pasten wie in Beispiel 1. Den 1000 Teilen Basispaste werden jeweils folgende Zusätze zugemischt:

| Beispiel | | |
|---|---|---|
| 2 | 10 Teile | $C_{12}H_{25}O(CH_2\text{-}CH_2O)_5H$ |
| 3 | 10 " | $C_{12}H_{25}O(CH_2\text{-}CH_2O)_5CO\text{-}CH_3$ |
| 4 | 10 " | $C_{13}H_{27}O(CH_2\text{-}CH_2O)_6H$ |
| 5 | 20 " | $C_{13}H_{27}O(CH_2\text{-}CH_2O)_6H$ |
| 6 | 10 " | $C_{13}H_{27}O(CH_2\text{-}CH_2O)_6CO\text{-}CH_3$ |
| 7 | 20 " | $C_{13}H_{27}O(CH_2\text{-}CH_2O)_6CO\text{-}CH_3$ |
| 8 (Vergleich) | 10 " | $C_9H_{19}\text{-}C_6H_4\text{-}O(CH_2\text{-}CH_2O)_{10}H$ |

Anwendungstechnische Prüfung:

Voraussetzung für die Gebrauchsfähigkeit der Abformmassen ist die Erfüllung der Anforderungen der Spezifikation ISO 4823 und der Spezifikation Nr. 19 der American Dental Association. Darüber hinaus werden der Benetzungs-Randwinkel als Nachweis für den Hydrophilie-Effekt sowie die Schabefestigkeit der Gipsprüfkörper als Nachweis für die Modellgipsverträglichkeit der vernetzten Abformmassen bestimmt. Die letzten beiden Methoden werden nachfolgend beschrieben:

Prüfung der Schabefestigkeit:

10 g Basis und 10 g Katalysatorpaste werden bei 23°C und 50 % rel. Luftfeuchte auf einem Anmischblock mittels Spatel innerhalb 30 Sekunden homogen gemischt und in einen Rillenblock (DIN 13913 Bild 3) gegeben. 15 Minuten nach Mischbeginn wird die vernetzte Siliconscheibe entformt und bei 23°C und 50% rel. Luftfeuchte 30 Minuten gelagert. Dann wird auf die Siliconscheibe ein im Durchmesser gleichgroßer Kunststoffring mit einer Höhe von 2 cm aufgelegt, mit einer wässrigen Gipsaufschlämmung (100 g Geostone®/23 g Wasser) aufgefüllt und mit einer Glasplatte abgedeckt. Eine Stunde nach Anmischung des Gipses unter Vakuum wird der Gipsprüfkörper von der Siliconplatte getrennt.

Zur Simulation des Schabens mit einem Modellierinstrument wird ein Stahlring mit einem Durchmesser von 4,3 mm und einer V-förmigen Schneide unter Belastungen von 70 g (Auflagegewicht 100 g) und 35 g (Auflagegewicht 50 g) bei einer Geschwindigkeit von 26 mm/min. senkrecht 4,8 mm weit über die Gipsoberfläche gezogen, so daß eine Spur in Form eines Kreisabschnittes entsteht. Die Breite der Spur (Sehne) wird unter schräg einfallendem Licht und bei Betrachtung im Stereomikroskop bei 20-facher Vergrößerung mit Hilfe eines Schraubenmikrometerokulars gemessen. Die Messungen sind mit einer maximalen Ungenauigkeit von 15 $\mu$m reproduzierbar.

Mittelwerte der Spurbreiten sind in $\mu$m angegeben. Jeder Mittelwert basiert auf je 3 Messungen an drei nebeneinander liegenden Spuren. Die mittlere und die maximale Standardabweichung von den Mittelwerten betragen 8 $\mu$m und 22 $\mu$m.

Es wird festgelegt, Produkte als schabfest einzustufen, wenn die Spurbreiten bei beiden Belastungen < 300 $\mu$m sind. Als "nicht schabfest" sollten solche Produkte charakterisiert werden, deren Spurbreite bei 100 g Auflagegewicht > 400 $\mu$m ist.

Messung des Benetzungs-Randwinkels:

20 g Basis- und 20 g Katalysatorpaste werden bei 23°C und 50 % rel. Luftfeuchte auf einen Anmischblock mittels Spatel innerhalb 30 Sekunden homogen gemischt in ein auf eine Glasplatte geklebtes Kunststoffareal von 9 x 5 x 0,5 cm gefüllt und mit einer Polyesterfolie abgedeckt. 15 Minuten nach Mischbeginn wurde die vernetzte Siliconplatte entformt. Nach 24-stündiger Lagerung bei 23°C und 50 % rel. Luftfeuchte wurde auf der dem Glas zugewandten Seite der Siliconplatte ein Wassertropfen aufgegeben und der Randwinkel bestimmt. Mittels des Video-Systems kann das dynamische Benetzungsverhalten von Flüssigkeiten auf Feststoffunterlagen direkt gemessen und ausgewertet werden. Während 15 Sekunden werden 49 Randwinkelmessungen durchgeführt. Als Anfangs-Randwinkel wurde der Randwinkelwert nach 1 Sekunde definiert (Mittelwert aus 3 Werten). Nach 13 Sekunden wird der Mittelwert des Randwinkels der letzten 10 Messungen angegeben (Zeitbereich ca. 11-15 Sekunden).

Ergebnisse:

| Bsp. | Hydrophilierungsmittel | Gipsprüfung Schabefestigkeit Spurbreite ($\mu$m) | Benetzungsrandwinkel | |
|---|---|---|---|---|
| | | | t = 1 sec. | t = 13 sec. |
| 1 | ohne | $322\pm17$ | $99\pm2$ | $100\pm3$ |
| 2 | 1 % $C_{12}H_{25}O(CH_2\text{-}CH_2O)_5H$ | $357\pm17$ | $74\pm4$ | $44\pm2$ |
| 3 | 1 % $C_{12}H_{25}O(CH_2\text{-}CH_2O)_5\text{-}CO\text{-}CH_3$ | $364\pm17$ | $77\pm3$ | $45\pm2$ |
| 4 | 1 % $C_{13}H_{27}O(CH_2\text{-}CH_2O)_6H$ | $331\pm13$ | $79\pm5$ | $62\pm3$ |
| 5 | 2 % $C_{13}H_{27}O(CH_2\text{-}CH_2O)_6H$ | $344\pm17$ | $58\pm2$ | $33\pm3$ |
| 6 | 1 % $C_{13}H_{27}O(CH_2\text{-}CH_2O)_6\text{-}CO\text{-}CH_3$ | $348\pm13$ | $72\pm3$ | $53\pm2$ |
| 7 | 2 % $C_{13}H_{27}O(CH_2\text{-}CH_2O)_6\text{-}CO\text{-}CH_3$ | $363\pm13$ | $61\pm4$ | $40\pm1$ |
| 8 | 1 % $C_9H_{19}\text{-}C_6H_4\text{-}O(CH_2\text{-}CH_2O)_{10}H$ | $490\pm14$ | $84\pm3$ | $48\pm4$ |

**Patentansprüche**

**Patentansprüche für folgende Vertragsstaaten : DE, DK, SE, AT, GR, CH, IT, FR, BE, NL, LU, GB**

1.  Additionsvernetzende Siliconabformmassen, enthaltend:
    (a) Organopolysiloxane mit mindestens zwei Vinylgruppen im Molekül,
    (b) gegebenenfalls Organopolysiloxane ohne reaktive Gruppen,
    (c) Organopolysiloxane mit zwei und mehr Si-H-Gruppen im Molekül,
    (d) Katalysator,
    (e) Füllstoffe sowie gegebenenfalls weitere übliche Zusatz,- Hilfs- und Farbstoffe,
    (f) einen mit 2 bis 10 Alkoxyeinheiten alkoxylierten $C_{10}$- bis $C_{16}$-Fettalkohol, der gegebenenfalls methyliert oder acyliert sein kann.
    dadurch gekennzeichnet, daß sie frei von Platinschwarz sind.

2.  Siliconabformmassen gemäß Anspruch 1, dadurch gekennzeichnet, daß die gerad- und verzweigtkettigen Fettalkohole (f) $C_{10}$- bis $C_{16}$-Alkohole sind, die mit 2 bis 10 Mol Ethylen- oder Propylenoxid umgesetzt und gegebenenfalls mit einer $C_2$- bis $C_4$-Monocarbonsäure verestert wurden.

3.  Siliconabformmassen nach Anspruch 1, dadurch gekennzeichnet, daß die gerad- oder verzweigtkettigen Fettalkohole (f) $C_{12}$-$C_{14}$-Fettalkohole sind, die mit 4 bis 7 Mol Ethylenoxid umgesetzt und gegebenenfalls mit Essigsäure verestert wurden.

4.  Siliconabformmassen gemäß Ansprüchen 1 und 2, dadurch gekennzeichnet, daß die Komponente (f) in Mengen von 0,3 bis 5,0 Gewichtsprozent, bezogen auf die Gesamtmischung zugesetzt werden.

**5.** Siliconabformmassen gemäß Ansprüchen 1 bis 4, zur Verwendung für die Zahn-, Schleimhaut- und Modellabformung.

**6.** Verfahren zur Herstellung Platinschwarz-freier additionsvernetzender Siliconabformmassen, enthaltend
(a) Organopolysiloxane mit mindestens zwei Vinylgruppen im Molekül,
(b) gegebenenfalls Organopolysiloxane ohne reaktive Gruppen,
(c) Organopolysiloxane mit zwei und mehr Si-H-Gruppen im Molekül,
(d) Katalysator,
(e) Füllstoffe sowie gegebenenfalls weitere übliche Zusatz,- Hilfs- und Farbstoffe,
(f) einen mit 2 bis 10 Alkoxyeinheiten alkoxylierten $C_{10}$- bis $C_{16}$-Fettalkohol, der gegebenenfalls methyliert oder acyliert sein kann.
dadurch gekennzeichnet, daß durch Vermischung, gegebenenfalls von Teilmengen, von Bestandteilen (a) bis (f) mit Ausnahme von (d) eine Basispaste A, durch Vermischung gegebenenfalls von Teilmengen von Bestandteilen (a) bis (f) mit Ausnahme von (c) eine Katalysatorpaste B hergestellt wird, und zur Herstellung der gebrauchsfertigen Abformmasse äquivalente Mengen A und B miteinander vermischt werden.

**Patentanspruch für folgenden Vertragsstaat : ES**

**1.** Verfahren zur Herstellung Platinschwarz-freier additionsvernetzender Siliconabformmassen, enthaltend
(a) Organopolysiloxane mit mindestens zwei Vinylgruppen im Molekül,
(b) gegebenenfalls Organopolysiloxane ohne reaktive Gruppen,
(c) Organopolysiloxane mit zwei und mehr Si-H-Gruppen im Molekül,
(d) Katalysator,
(e) Füllstoffe sowie gegebenenfalls weitere übliche Zusatz,- Hilfs- und Farbstoffe,
(f) einen mit 2 bis 10 Alkoxyeinheiten alkoxylierten $C_{10}$- bis $C_{16}$-Fettalkohol, der gegebenenfalls methyliert oder acyliert sein kann
dadurch gekennzeichnet, daß durch Vermischung, gegebenenfalls von Teilmengen, von Bestandteilen (a) bis (f) mit Ausnahme von (d) eine Basispaste A, durch Vermischung gegebenenfalls von Teilmengen von Bestandteilen (a) bis (f) mit Ausnahme von (c) eine Katalysatorpaste B hergestellt wird, und zur Herstellung der gebrauchsfertigen Abformmasse äquivalente Mengen A und B miteinander vermischt werden.

**Claims**

**Claims for the following Contracting States : DE, DK, SE, AT, GR, CH, IT, FR, BE, NL, LU, GB**

**1.** Addition-crosslinking silicone impression compounds containing:
(a) organopolysiloxanes containing at least two vinyl groups in the molecule,
(b) optionally organopolysiloxanes containing no reactive groups,
(c) organopolysiloxanes containing two or more SiH groups in the molecule,
(d) a catalyst,
(e) fillers and optionally further conventional additives, auxiliaries and dyes,
(f) a $C_{10}$- to $C_{16}$-fatty alcohol which has been alkoxylated by 2 to 10 alkoxy units and may optionally be methylated or acylated
characterized in that they contain no platinum black.

**2.** Silicone impression compounds according to Claim 1, characterized in that the straight-chain and branched fatty alcohols (f) are $C_{10}$- to $C_{16}$-alcohols which have been reacted with from 2 to 10 mol of ethylene oxide or propylene oxide and have optionally been esterified by means of a $C_2$- to $C_4$-monocarboxylic acid.

**3.** Silicone impression compounds according to Claim 1, characterized in that the straight-chain or branched fatty alcohols (f) are $C_{12}$-$C_{14}$-fatty alcohols which have been reacted with from 4 to 7 mol of ethylene oxide and have optionally been esterified by means of acetic acid.

**4.** Silicone impression compounds according to Claims 1 and 2, characterized in that component (f) is added in amounts of 0.3 to 5.0 per cent by weight, based on the total mixture.

**5.** Silicone impression compounds according to Claims 1 to 4, for use for casting teeth, mucous membranes and models.

**6.** Process for the preparation of platinum black-free addition-crosslinking silicone impression compounds containing

(a) organopolysiloxanes containing at least two vinyl groups in the molecule,

(b) optionally organopolysiloxanes containing no reactive groups,

(c) organopolysiloxanes containing two or more SiH groups in the molecule,

(d) a catalyst,

(e) fillers and optionally further conventional additives, auxiliaries and dyes,

(f) a $C_{10}$- to $C_{16}$-fatty alcohol which has been alkoxylated by 2 to 10 alkoxy units and may optionally be methylated or acylated

characterized in that a base paste A is prepared by mixing all or some of each of constituents (a) to (f), with the exception of (d), a catalyst paste B is prepared by mixing all or some of each of constituents (a) to (f), with the exception of (c), and equivalent amounts of A and B are mixed with one another to prepare the ready-for-use impression compound.

**Claim for the following Contracting State : ES**

**1.** Process for the preparation of platinum black-free addition-crosslinking silicone impression compounds containing

(a) organopolysiloxanes containing at least two vinyl groups in the molecule,

(b) optionally organopolysiloxanes containing no reactive groups,

(c) organopolysiloxanes containing two or more SiH groups in the molecule,

(d) a catalyst,

(e) fillers and optionally further conventional additives, auxiliaries and dyes,

(f) a $C_{10}$- to $C_{16}$-fatty alcohol which has been alkoxylated by 2 to 10 alkoxy units and may optionally be methylated or acylated

characterized in that a base paste A is prepared by mixing all or some of each of constituents (a) to (f), with the exception of (d), a catalyst paste B is prepared by mixing all or some of each of constituents (a) to (f), with the exception of (c), and equivalent amounts of A and B are mixed with one another to prepare the ready-for-use impression compound.

**Revendications**

**Revendications pour les Etats contractants : DE, DK, SE, AT, GR, CH, IT, FR, BE, NL, LU, GB**

**1.** Masses d'empreintes en silicones, réticulant par addition, contenant :

(a) des organopolysiloxanes à au moins deux groupes vinyle par molécule,

(b) le cas échéant des organopolysiloxanes sans groupe réactif,

(c) des organopolysiloxanes à deux groupes Si-H ou plus dans la molécule,

(d) un catalyseur,

(e) des matières de charge et le cas échéant d'autres additifs, produits auxiliaires et colorants usuels,

(f) un alcool gras en $C_{10}$-$C_{16}$ alcoxylé par 2 à 10 motifs alcoxy et qui peut le cas échéant être méthylé ou acylé,

caractérisées en ce qu'elles sont exemptes de noir de platine.

**2.** Masses d'empreintes en silicones selon la revendication 1, caractérisées en ce que les alcools gras (f), à chaîne droite ou ramifiée, sont des alcools en $C_{10}$-$C_{16}$ condensés avec 2 à 10 mol d'oxyde d'éthylène ou de propylène et le cas échéant estérifiés par un acide monocarboxylique en $C_2$-$C_4$.

**3.** Masses d'empreintes en silicones selon la revendication 1, caractérisées en ce que les alcools gras (f), à chaîne droite ou ramifiée, sont des alcools gras en $C_{12}$-$C_{14}$ condensés avec 4 à 7 mol d'oxyde d'éthylène et éventuellement estérifiés par l'acide acétique.

**4.** Masses d'empreintes en silicones selon les revendications 1 et 2, caractérisées en ce que le composant (f) est ajouté en quantité de 0,3 à 5,0 % du poids du mélange total.

5. Masses d'empreintes en silicones selon les revendications 1 à 4, pour l'utilisation pour l'empreinte de dents, de gencives et le moulage de modèles.

6. Procédé de préparation de masses d'empreintes en silicones réticulant par addition, exemptes de noir de platine, contenant

(a) des organopolysiloxanes à au moins deux groupes vinyle par molécule,

(b) le cas échéant des organopolysiloxanes sans groupe réactif,

(c) des organopolysiloxanes à deux groupes Si-H ou plus par molécule,

(d) un catalyseur,

(e) des matières de charge et le cas échéant d'autres additifs, produits auxiliaires et colorants usuels,

(f) un alcool gras en $C_{10}$-$C_{16}$ alcoxylé par 2 à 10 motifs alcoxy et qui peut le cas échéant être méthylé ou acylé,

caractérisé en ce que l'on prépare par mélange, éventuellement par portions, des composants (a) à (f) à l'exception de (d), une pâte de base A, on prépare par mélange, éventuellement par portions, des composants (a) à (f) à l'exception de (c), une pâte de catalyseur B, et, pour la préparation de la masse d'empreinte prête à l'emploi, on mélange entre elles des quantités équivalentes de A et B.

**Revendication pour l'Etat contractant : ES**

1. Procédé de préparation de masses d'empreintes en silicones réticulant par addition et exemptes de noir de platine, qui contiennent

(a) des organopolysiloxanes à au moins deux groupes vinyle par molécule,

(b) le cas échéant des organopolysiloxanes sans groupe réactif,

(c) des organopolysiloxanes à deux groupes Si-H ou plus par molécule,

(d) un catalyseur,

(e) des matières de charge et le cas échéant d'autres additifs, produits auxiliaires et colorants usuels,

(f) un alcool gras en $C_{10}$-$C_{16}$ alcoxylé par 2 à 10 motifs alcoxy, et qui peut le cas échéant être méthylé ou acylé,

caractérisé en ce que l'on prépare par mélange, éventuellement par portions, des composants (a) à (f) à l'exception de (d), une pâte de base A, on prépare par mélange, éventuellement par portions, des composants (a) à (f) à l'exception de (c), une pâte de catalyseur B, et, pour la préparation de la masse d'empreinte prête à l'emploi, on mélange entre elles des quantités équivalentes de A et B.